# EUROPEAN PATENT SPECIFICATION

(11) **EP 3 903 859 B1**
(45) Date of publication and mention of the grant of the patent: **24.09.2025**
(21) Application number: 19906350.4
(22) Date of filing: 27.12.2019
(51) Int. Cl.: A61M 5/20, A61M 5/30, A61M 5/24

(54) **NEEDLELESS INJECTOR**
NADELLOSER INJEKTOR
INJECTEUR SANS AIGUILLE

(30) Priority: 27.12.2018 JP 2018245623
(43) Date of publication of application: 03.11.2021
(73) Proprietor: Daicel Corporation, Osaka 530-0011 (JP)
(72) Inventor: HIRAYAMA, Yayoi, Tokyo 108-8230 (JP); IGA, Hiromitsu, Tokyo 108-8230 (JP); YAMAMOTO, Yuzo, Tokyo 108-8230 (JP)
(74) Representative: Grünecker Patent- und Rechtsanwälte PartG mbB
(86) International application number: PCT/JP2019/051555
(87) International publication number: WO 2020/138475

(56) References cited:
- WO-A1-2015/067747
- WO-A1-2015/067747
- WO-A1-2018/093622
- WO-A1-2018/093622
- JP-A- 2003 310 758
- JP-A- 2003 310 758
- JP-A- 2004 521 681
- JP-A- 2004 521 681
- JP-A- 2016 198 328
- JP-A- 2016 198 328
- JP-A- 2017 023 813
- JP-A- 2017 023 813
- US-A1- 2017 049 967
- US-A1- 2018 056 002
- US-A1- 2018 056 002
- US-B1- 6 193 698
- US-B1- 6 193 698

## Description

### Technical Field

The present disclosure relates to a needleless injector that ejects an intended injection substance to a target region without using an injection needle.

### Background Art

An example of a device that ejects a liquid chemical to a target region such as an organism includes a needleless injector using no injection needle which has been attracting attention in terms of usability and sanitation, and thus has been actively developed recently. In general, there has been implemented a needleless injector having a configuration in which a liquid chemical pressurized by a driving source such as compressed gas and a spring is ejected to a target region and the liquid chemical is administered to an inside of the target region through use of the kinetic energy of the liquid chemical. Such needleless injectors are known from JP 2003 310 758 A and JP 2017 023 813. Furthermore, US 2017/049967 A1 shows a needleless injector according to the preamble of claim 1.

Various operation mechanisms for activating such a needleless injector have been disclosed. For example, Patent Document 1 discloses an operation mechanism for safely using a needleless injector. With the needleless injector, due to its feature, the liquid chemical is vigorously ejected to the outside. Thus, the liquid chemical is not preferably ejected by an unintentional operation on the needleless injector. Thus, the needleless injector of Patent Document 1 includes, in addition to a switch for activating an igniter provided on a side surface of a housing of the needleless injector, a standby switch protruding on a distal end surface of the housing. This standby switch is formed in its protr uding state, by biasing it with a spring inside the housing. When an ejection port in the distal end surface of the housing is brought into contact with the target region for using the needleless injector, the standby switch is pushed in such that the needleless injector enters a standby state, and the switch for activating the igniter becomes operable.

### Citation List

### Patent Document

Patent Document 1: JP 2015-150401 A

### Summary of Invention

### Technical Problem

In general, the needleless injector is used with its ejection port placed in a predetermined state with respect to the target region (such as, for example, a state where the ejection port is in contact with the target region or a state where the ejection port is facing the target region), and the liquid chemical or the like ejected can be expected to behave as designed in the target region. In view of this, according to a related art, the standby switch is provided in addition to the switch for activating the igniter, and the igniter can be prevented from being activated in a state where the ejection port of the needleless injector is not in contact with the target region. Thus, the needleless injector has an operation mechanism requiring two stage operation, for the sake of improved safety.

Unfortunately, the operation on the needleless injector by the user or the state of the target region is not always the same. The reaction force received by the standby switch from the target region might varies due to a difference between individuals in how the housing of the needleless injector comes into contact with the target region or a difference in elasticity of the target region or the like. Thus, even when the user operates the needleless injector to make the ejection port come into contact with the target region for placing the needleless injector in the standby state by pushing in the standby switch, the standby state might not be favorably achieved. As a result, a stable operation of the needleless injector might be hindered.

In view of the above problem, an object of the present disclosure is to provide an operation mechanism for a needleless injector with which improved safety and stable operation may both be achieved.

### Solution to Problem

To solve the problem described above, a needleless injector according to claim 1 is provided. In a needleless injector according to the present disclosure, two switches are used as switches for activating a driving part related to imparting of ejection energy such that the operation is performed in two stages, and a mode of arrangement of the switches on the housing is adjusted for achieving the safety and the stable operation of the needleless injector.

Specifically, the present disclosure relates to a needleless injector that ejects an intended injection substance to a target region without using an injection needle, the needleless injector comprising: a housing part that includes an accommodating space in which the intended injection substance is accommodated, and defines a flow path through which the intended injection substance is ejected to the target region; a driving part that imparts an ejection energy for ejecting the intended injection substance; a pressurizing unit that, upon being imparted with the ejection energy, pressurizes the intended injection substance accommodated in the accommodating space; an injector enclosure that has a tubular shape, incorporates at least one of the housing part, the driving part, or the pressurizing unit, and is configured to be capable of being held by one hand of the user; a first switch and a second switch for activation of the driving part, the first switch and the second switch being provided to the injector enclosure; and a control unit that controls the activation of the driving part, via a predetermined operation on the first switch and the second switch. The first switch is arranged to be slidable in a predetermined direction different from a contact direction of a palm of the user, in a first region on a side surface that is a part of a surface of the injector enclosure positioned on side of the user, the first region being covered by the palm of the user in a holding state of the user, and the second switch has an upper side surface with at least a part inclined toward the side of the user to be visible from the user in a holding state of the user, and is arranged on an upper side of the injector enclosure to be able to be pressed.

In the needleless injector described above, the driving part imparts the ejection energy, and thus the intended injection substance is ejected to the target region. **In** the present application, "ejection" is achieved by pressurizing the intended injection substance in the housing part with the pressurizing unit using the ejection energy imparted by the driving part, to allow the intended injection substance to flow through the flow path in the housing part.

Here, as the intended injection substance ejected from the needleless injector, predetermined substances including a component expected to have effects in the target region or a component expected to exert a predetermined function in the target region can be exemplified. Thus, the intended injection substance may have any physical form, as long as the ejection by the ejection energy as described above can at least be performed. For example, the intended injection substance may be dissolved in liquid, or may be simply mixed with liquid without dissolving therein. As one example, the predetermined substance to be sent includes vaccine for intensifying an antibody, a protein for cosmetic enhancement, a cultured cell for hair regeneration, and the like, and is included in a liquid medium in an ejectable manner to formthe intended injection substance. Note that the medium is preferably a medium that does not hinder the above-mentioned effect and function of the predetermined substance in a state of being injected into the target region. As another method, the medium may be a medium that exerts the above-mentioned effect and function by acting together with the predetermined substance in the state of being injected into the target region.

The intended injection substance ejected needs to penetrate the surface of the target region such that the intended injection substance is ejected from the needleless injector to the target region to be delivered into the inside thereof. Thus, at an ejection initial state, the intended injection substance needs to be ejected to the target region at a relatively high speed. In view of this point, as an example, the driving part preferably imparts the ejection energy using a combustion product discharged by combustion of an ignition charge. Note that, as the ignition charge, there may be employed any one of an explosive containing zirconium and potassium perchlorate, an explosive containing titanium hydride and potassium perchlorate, an explosive containing titanium and potassium perchlorate, an explosive containing aluminum and potassium perchlorate, an explosive containing aluminum and bismuth oxide, an explosive containing aluminum and molybdenum oxide, an explosive containing aluminum and copper oxide, an explosive containing aluminum and iron oxide, or an explosive composed of a combination of a plurality of the above explosives. As characteristics of the above-mentioned ignition charge, the combustion product is gas at high temperatures but does not include a gas component at a room temperature, and hence the combustion product is condensed immediately after the ignition. As a result, the driving part can impart the ejection energy in an extremely short period of time. In addition, the driving part may utilize electrical energy of a piezoelectric element or the like or mechanical energy of a spring or the like as the ejection energy instead of the ejection energy caused by the combustion of the ignition charge, and may generate the ejection energy by appropriately combining these forms of energy.

The injector enclosure formed to enable the user to hold the needleless injector with one hand when using the needleless injector is provided with the first switch and the second switch for activating the needleless injector. The control unit determines the content of operation on each switch, and activates the driving part when an operation suitable for the activation of the needleless injector is performed, and the intended injection substance is ejected from the needleless injector. Thus, the operation for activating the needleless injector is performed in two stages using the two switches, whereby the safety of the needleless injector is guaranteed.

The needleless injector described above has the first switch and the second switch arranged at positions different from each other, and the content of the operation for activating the needleless injector differs between the switches. Specifically, the first switch is a sliding switch that is arranged in the first region in the side surface of the injector enclosure. The first region is the region covered by the palm of the user attempting to hold the injector enclosure with one hand. Thus, when the user holds the injector enclosure, the palm comes into contact with the first switch from the above. The contact direction, that is, the direction in which the palm approaches the first switch is different from the sliding direction that is an operation direction of the sliding first switch. Thus, the first switch is favorably prevented from receiving an unintended sliding operation due to an operation of holding the injector enclosure by the user. To perform the sliding operation on the first switch, for example, the user has to move the part of the palm temporarily away from the first region such that some of the fingers rest on the injector enclosure with the first switch being accessible by a remaining finger.

The second switch is a pressing switch arranged on the upper side of the injector enclosure. At least a part of the upper side surface of the second switch is formed to be inclined toward the side of the user. The at least a part of the upper side surface of the second switch is a portion that may be a pressing portion. With the pressing portion formed as described above, the user who holds the injector enclosure with one hand can easily visually recognize the pressing portion of the second switch. This configuration enables the user to clearly recognize the presence of the second switch that is a pressing switch, and thus leads to a stable operation of the needleless injector. Furthermore, the way the user holds the injector enclosure is defined, and the holding can be performed with high reproducibility, whereby stable injection is achieved.

The control unit receives the content of the operation by the user on the first switch and the second switch thus configured, determines whether the operation is appropriate, and controls the activation of the driving part. Thus, with the above described configuration in which the two switches requiring operations of different contents are arranged at different positions such that the operation is performed in two stages of contents and the suitable operation on the first switch and the second switch is guaranteed, the improvement of safety and stable operation of the needleless injector can both be achieved.

In the needleless injector described above, the second switch may be arranged in a second region in the upper side surface of the injector enclosure, the second region being configured to be inclined, similarly to the at least a part of the upper side surface of the second switch, toward the side of the user. With the second switch thus arranged in the second region, the second region can be visually recognized in a wide range together with the pressing portion of the second switch by the user who holds the injector enclosure with one hand, whereby the second switch can be expected to be more stably operated.

In the needleless injector described above, the second switch in the second region may be arranged on a virtual straight line that is an extension, along the surface of the injector enclosure, of a slide straight line of the first switch along the predetermined direction, or in vicinity of the virtual straight line. With this configuration, the user who holds the injector enclosure with one hand can smoothly and continuously perform the sliding operation on the first switch and the pressing operation on the second switch using a finger of the one hand for example. This contributes greatly to improving the operability of the needleless injector by the user. The slide straight line described above is a straight line defined by the sliding direction of the sliding first switch.

In the needleless injector described above, the control unit may be configured to activate the driving part in response to a predetermined pressing operation on the second switch performed after a predetermined sliding operation on the first switch by the user. Specifically, the control unit allows the driving part to be activated when the predetermined sliding operation is performed on the first switch and then the predetermined pressing operation is performed on the second switch. In this case, the predetermined sliding operation is an operation for placing the needleless injector in a state in which the preparation for ejection has been completed (standby state). The predetermined pressing operation is an operation of executing the ejection of the intended injection substance from the needleless injector in the standby state. With this configuration, the user can perform the operations for executing the ejection sequentially, while accurately recognizing the status of the needleless injector. Thus, an erroneous operation by the user can be favorably prevented. Operations other than those described above can be used for operating the first switch and the second switch for allowing the driving part to be activated.

The predetermined sliding operation may be an operation of sliding the first switch in the predetermined direction and maintaining the sliding state for a predetermined period of time. Requiring the user to thus maintain the sliding state for a predetermined period of time to place the needleless injector in the standby state means that the activation of the needleless injector is implemented under the clear intention of the user. Thus, erroneous ejection of the intended injection substance with the needleless injector unintentionally placed in the standby state can be prevented, whereby a high level of safety can be maintained. The predetermined period of time may be determined as appropriate based on a balance between the operability of the needleless injector by the user and the safety guaranteed.

In the needleless injector described above, the driving part may be an ignition device that generates the ejection energy through combustion of an ignition charge upon being supplied with ignition current, a power cable for supplying the ignition current from a power supply to the ignition device may extend from the injector enclosure, and the injector enclosure may be configured to have the power cable extending toward a direction away from the user in a holding state of the user with the first switch facing toward the user. The ignition device is an electrical igniter, and thus the ignition current needs to be supplied to the ignition device to impart the ejection energy. When the ignition current is fed in from the outside of the needleless injector through the power cable, the power is in a state of extending from the injector enclosure. Thus, the movement of the needleless injector is limited by the power cable. Therefore, the power cable compromises the operability of the needleless injector by the user. Still, with the power cable extending in a direction away from the user as described above, such an impact of the power cable can be reduced as much as possible.

More preferably, the injector enclosure has a side surface, on a side opposite to the first switch with the second switch provided in between, provided with a connector to which the power cable is connected, and the connector is attached to the injector enclosure to make the power cable extend along an inclined surface defined by the at least a part of the upper side surface of the second switch. With this configuration, the level of interference of the power cable to the user who holds the injector enclosure with one hand can be lowered as much as possible. The connector may be configured to enable the power cable to be detachably attached.

### Advantageous Effects of Invention

An operation mechanism for a needleless injector with which improved safety and stable operation may both be achieved can be provided.

### Brief Description of Drawings

FIG. 1 is a diagram illustrating a schematic configuration of a needleless injector.
FIG. 2 is a first cross-sectional view of a needleless injector.
FIG. 3 is a second cross-sectional view of the needleless injector.
FIG. 4 is a diagram illustrating a configuration of a housing of the needleless injector.
FIG. 5 is a diagram illustrating a schematic configuration of an injector assembly incorporated in the needleless injector.
FIG. 6 is a diagram illustrating a schematic configuration of an actuator incorporated in the needleless injector.
FIG. 7 is a diagram illustrating a schematic configuration of a piston incorporated in the needleless injector.
FIG. 8 is a diagram illustrating a schematic configuration of an attachment incorporated in the needleless injector.
FIG. 9 is a diagram illustrating a schematic configuration of a plunger rod and plunger incorporated in the needleless injector.
FIG. 10 is a diagram illustrating a schematic configuration of a container incorporated in the needleless injector.
FIG. 11 is a flowchart illustrating an operation procedure for the needleless injector.

### Description of Embodiments

With reference to the drawings, a needleless injector 1 according to an embodiment of the present disclosure (herein, simply referred to as "injector") is described below. The injector 1 is a needleless injector that implements injection by ejecting an ejection solution, which corresponds to an intended injection substance in the present application, to a target region through use of a combustion energy of an explosive, that is, a device that injects the ejection solution to the target region without using an injection needle.

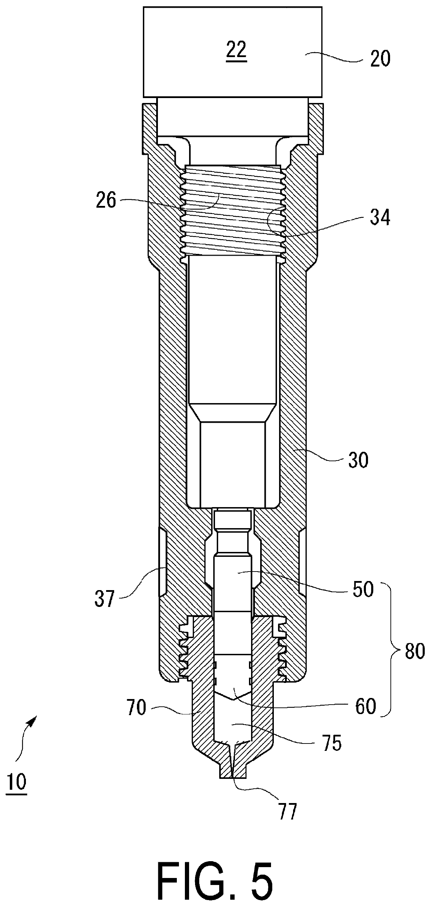

Note that each of the configurations, combinations thereof, and the like in each embodiment are an example, and various additions, omissions, substitutions, and other changes may be made as appropriate without departing from the spirit of the present disclosure. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### Configuration of Injector 1

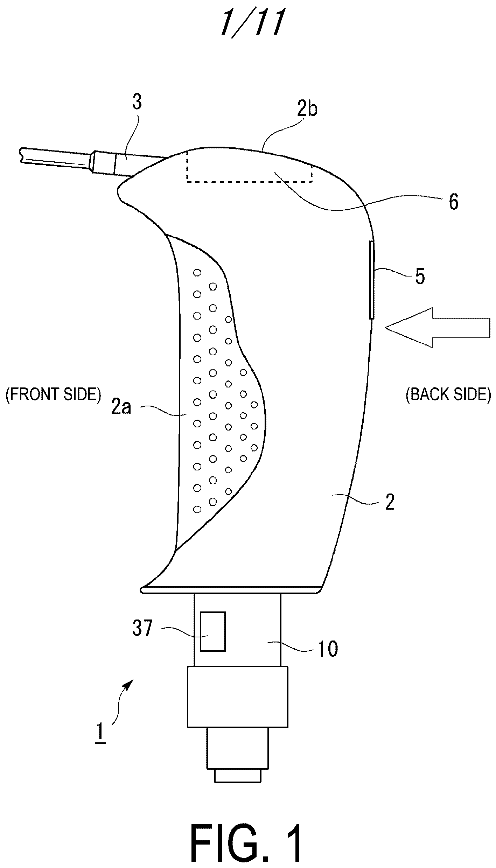

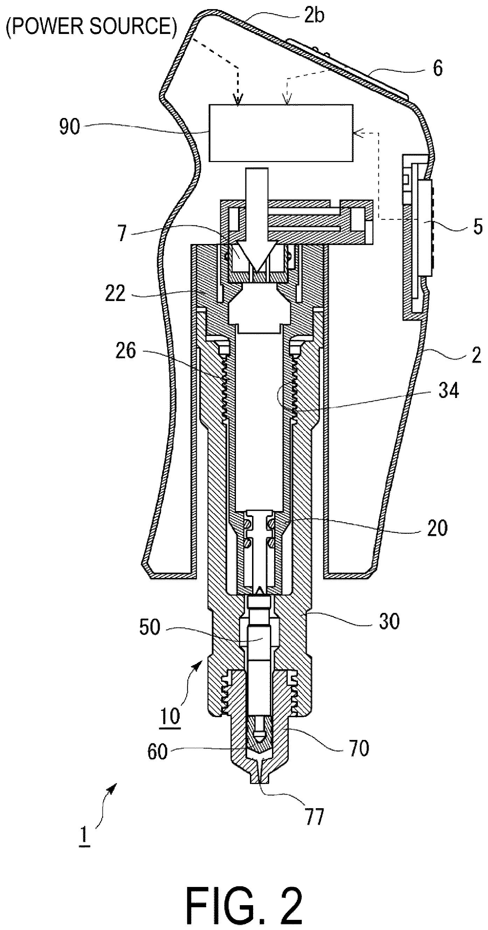

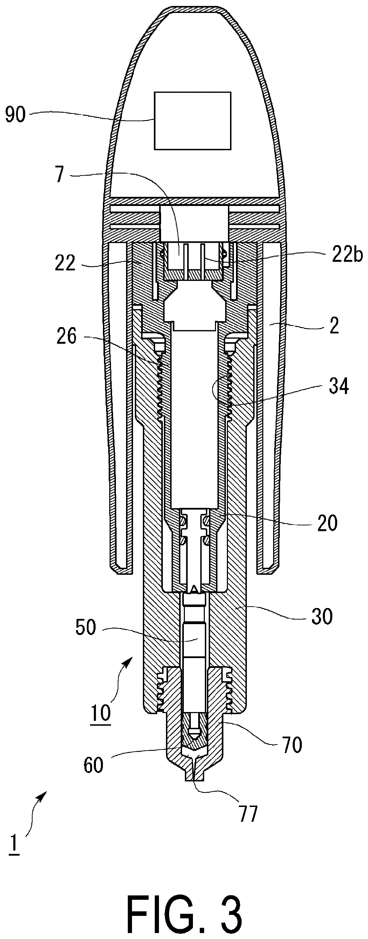

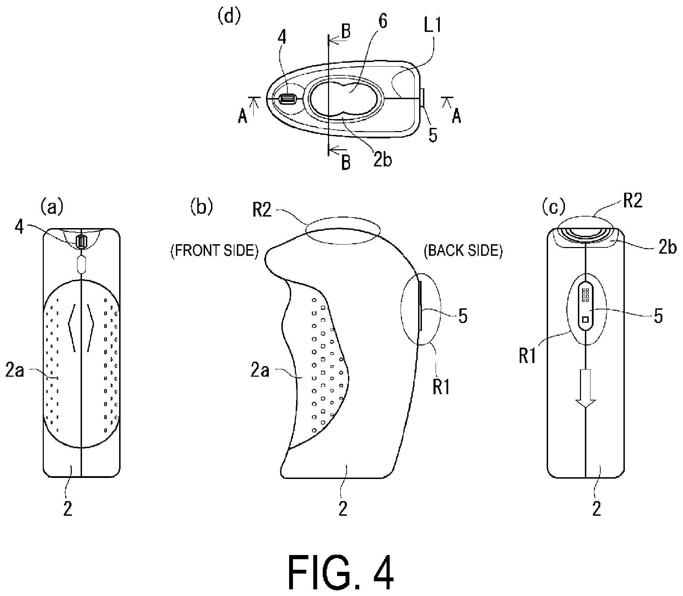

Here, FIG. 1 is a diagram schematically illustrating the appearance of the injector 1. FIG. 2 is a first cross-sectional view of the injector 1, which is an AA cross section in FIG. 4, described below. FIG. 3 is a second cross-sectional view of the injector 1, a BB cross section in FIG. 4 described below. The BB cross section is orthogonal to the AA cross section. Note that FIG. 4 is a diagram illustrating a configuration of a housing 2 that is a part of the injector 1. Here, the injector 1 is formed with the injector assembly 10 attached to the housing 2 (injector enclosure). A power cable 3 for supplying drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed, rather than being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid as the medium of the ejection solution is only required to be a substance suitable for administering the predetermined substance as exemplified above to the target region, and may be aqueous or oleaginous, which is not limited. Further, viscosity of the liquid as the medium is not particularly limited as long as the predetermined substance can be ejected by the injector 1.

In the injector 1, the injector assembly 10 is freely attachable to and detachable from the housing 2. An accommodating space 75 (see FIG. 5) formed between a container 70 and a plunger 80 in the injector assembly 10 is filled with ejection solution during a preparation stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 is configured to be capable of being held and operated by one hand of the user. In this context, the housing 2 will be described with reference to FIG. 4. In FIG. 4, (a) illustrates the outer appearance of the housing 2 as viewed from the front side, (b) illustrates the outer appearance of the housing 2 as viewed from one side, (c) illustrates the outer appearance of the housing 2 as viewed from the back side, and (d) illustrates the outer appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). The "front side" and "back side" as illustrated in FIG. 4(b) respectively match the "front side" and "back side" illustrated in FIG. 1. Thus, when the user holds the housing 2 with one hand, fingertips rest on the front side of the housing 2 which is the distal side, and the wrist is in the vicinity of the back side of the housing 2 which is the proximal side. The "upper side" is a portion of the injector 1 on the base end side.

Considering such a way of holding by the user, the grip portion 2a is provided at a front side portion of the housing 2 such that the user can easily rest user's fingertips thereon. The grip portion 2a is provided with a plurality of dimples, making the user's fingertips even easier to be rested thereon. Furthermore, the grip portion 2a has gentle recesses and protrusions on the front side of its outer shell (see (b) in FIG. 4) such that the user's forefinger and middle finger can be easily rested thereon, for the sake of more stable holding of the housing 2 by the user.

Further, the housing 2 is provided with a first switch 5 and a second switch 6 that are two operating switches for operating the injector 1. The first switch 5 and the second switch 6 are connected to a control unit 90, formed by a microcomputer. The control unit 90 controls the supply of ignition current to the igniter 22 based on a signal transmitted from each switch, thereby controlling an operation of the injector 1. The first switch 5 is a sliding switch provided in a first region R1 in a side surface on the back side of the housing 2, the sliding direction of which being an upward and downward direction of the housing 2 (direction between the distal end and the base end, as indicated by a white arrow in FIG. 4(c)). The first region R1 is a region covered by the palm (a portion around the base of the thumb) of the user who attempts to hold the housing 2 with one hand. Thus, when the user holds the housing 2, the palm of the hand comes into contact with the first switch 5 while covering the first switch 5 from the above. The contact direction of the palm, that is, a direction in which the palm approaches the first switch 5 (a direction indicated by a white arrow in FIG. 1) is a direction different from the sliding direction (a direction toward the distal end of the injector 1) that is an operation direction of the sliding first switch. When the user performs a sliding operation on the first switch 5, user's palm is partially separated from the first region R1 temporarily with some of the fingers (the forefinger or the middle finger for example) resting on the housing 2 while enabling a remaining finger (the thumb for example) access the first switch 5, and the sliding operation can be performed on the first switch 5 using the remaining finger.

The first switch 5 is constantly biased in the upward direction. When the user continuously slides the first switch 5 downward (toward the distal end side) for a predetermined period of time against the biasing force, the control unit 90 detects the sliding state of the first switch 5 and can place the injector 1 in a standby state. The standby state is a state in which preparation for the injector 1 to eject the ejection solution has been completed. When a user makes an additional operation (an operation on the second switch 6 described later) in this state, the ejection is implemented. The first switch 5 configured as described above is an operation switch for placing the injector 1 in the standby state. The contact direction of the palm of the user to hold the housing 2 is different from the operation direction of the first switch 5. Thus, the first switch 5 can be prevented from receiving an unintentional sliding operation by the user who holds the housing 2.

The second switch 6 is a pressing switch provided in a second region R2 including an inclined surface 2b on the upper side of the housing 2. The user can press the second switch 6 in a direction toward the inner side of the housing 2. The second region R2 is a region that is exposed without being covered by the palm of the user who holds the housing 2. The second switch 6 has an upper side surface inclined toward the side of the user, together with the inclined surface 2b in the second region R2. Alternatively, the upper side surface of the housing 2 may form the inclined surface without the upper side surface of the second switch 6 inclining. When at least the upper side surface of the second switch 6 thus forms the inclined surface, the user who holds the housing 2 with one hand can more easily visually recognize the upper side surface, which may be the pressing portion of the second switch 6, and can perform the pressing operation on the second switch 6 in a natural holding state. The upper side surface of the second switch 6 is thus more easily recognizable by the user and the second switch 6 is thus able to be pressed in the natural holding state, which enables the user to stably operate the injector 1 while clearly recognizing the presence of the second switch such that erroneous operation can be prevented, and thus leads to more reproducible administration.

As described above, the control unit 90 supplies an ignition current to the igniter 22 in response to the pressing operation on the second switch 6 while the injector 1 is in the standby state as a result of the operation on the first switch 5 described above. As a result, the injector 1 ejects the ejection solution. In view of this, the second switch 6 may be regarded as the operation switch of the injector 1 for determining the ejection of the ejection solution. Thus, the configuration in which the upper side surface of the second switch 6 is formed by an inclined surface to be more visible to the user as described above may be regarded as an extremely effective configuration in terms of stable operation of the injector 1.

Now, a relative positional relationship between the first switch 5 and the second switch 6 will be described. As illustrated in FIG. 4, in the second region R2, the second switch 6 is arranged on a virtual straight line L1, which is a straight line as a virtual extension of a slide straight line defined by the sliding direction of the first switch 5 (corresponding to the straight line extending in a direction indicated by a white arrow in FIG. 4(c)), along the surface of the housing 2. As described above, the first region R1 is the region covered by the palm of the user who attempts to hold the housing 2 with one hand, while the second region R2 is the area that is not covered by the user's palm. With the first switch 5 and the second switch 6 arranged at the relative positions as described above, the user who has performed the sliding operation on the first switch 5 using a certain finger can continuously perform the pressing operation on the second switch 6 smoothly using the same finger, while tightly holding the housing 2 with user's palm. This contributes greatly to improving the operability of the injector 1 by the user. The second switch 6 does not necessarily need to be arranged on the virtual straight line L1, and may be arranged in the vicinity of the virtual straight line L1, as long as the pressing operation on the second switch 6 can be smoothly performed after the sliding operation on the first switch 5. The first switch 5 and the second switch 6 may be arranged in a center portion of the housing 2 in the width direction or in the vicinity thereof as illustrated in FIGS. 4(a) and 4(c), and the favorable operability can be achieved while taking a difference in the dominant hand of the user into consideration.

A connector 4 to which the power cable 3 is connected is provided on the front side of the inclined surface 2b on the upper side surface of the housing 2. In the present embodiment, the connector 4 is a USB connector, and the power cable 3 is freely attachable to and detachable the housing 2. Alternatively, the power cable 3 may be a cable that is not detachable from the housing 2. FIG. 1 illustrates a state in which the power cable 3 is connected to the housing 2 via the connector 4. As illustrated, the power cable 3 extends toward the front side from the housing 2 of the injector 1. In other words, the user who holds the housing 2 of the injector 1 with one hand is positioned on the back side of the housing 2, whereas the power cable 3 extends toward the front side from the housing 2, that is, in a direction away from the user who holds the housing 2. Thus, the operation on the injector 1 by the user can smoothly progress without the power cable 3 hindering the operation by the user of the injector 1.

As illustrated in FIG. 1, the power cable 3 connected to the connector 4 extends, in a portion around the connection portion between these, in a direction substantially along the inclination direction of the inclined surface 2b of the housing 2. In other words, in FIG. 1, the power cable 3 is directed upward on the front side. With such a configuration, a relatively large space can be secured between the extending power cable 3 and the housing 2. Thus, the level interference of the power cable 3 to the hand of the user who holds the housing 2 can be lowered as much as possible. It is a matter of course that the power cable 3 may extend in a direction other than that in the mode described above. Still, the extending direction is preferably determined while taking the level of interference to the user's hand into consideration.

Note that, as described above, in the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator 20, an attachment 30, the container 70, and the plunger 80. How the injector assembly 10 is assembled will be described below.

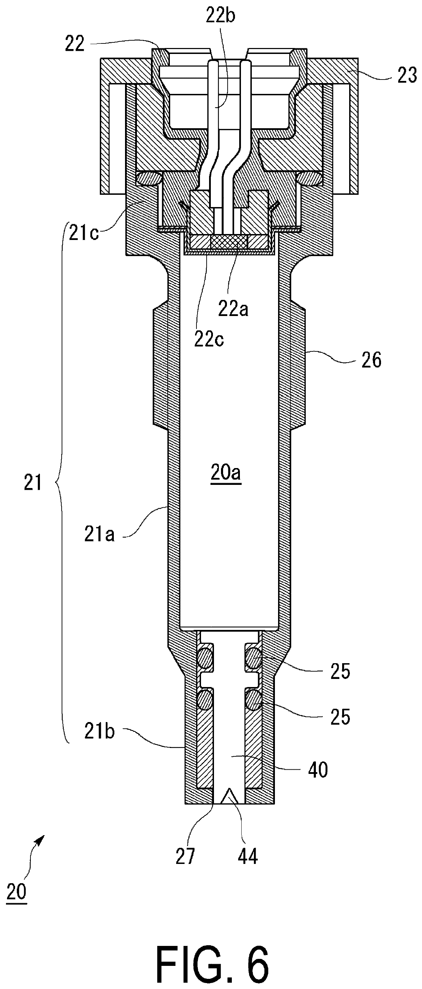

First of all, the actuator 20 will be described with reference to FIG. 6. The actuator 20 has a body 21 formed in a tubular shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. The igniter 22, which is an electric igniter that generates energy for ejection through combustion of an ignition charge 22a, is attached to the base end portion 21c of the body 21 via a cap 23. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

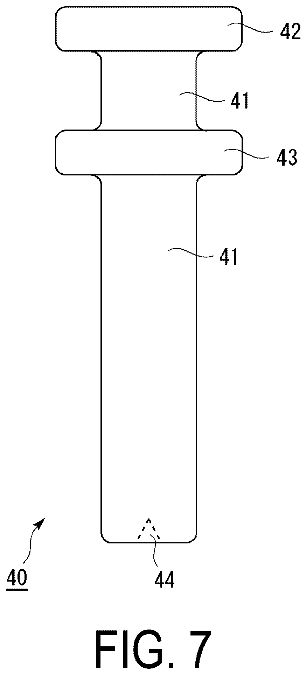

The internal space of the center portion 21a of the body 21 serves as a combustion chamber 20a into which a combustion product is discharged from the igniter 22. Furthermore, a male thread portion 26 is formed in a part of the outer surface of the center portion 21a. The male thread portion 26 is configured to mate with a female thread portion 32 of the attachment 30 described below. The effective lengths of the male thread portion 26 and the female thread portion 32 are determined to guarantee sufficient coupling force therebetween. The internal space of the distal end portion 21b adjacent to the center portion 21a is formed in a cylindrical shape in which a piston 40 is slidably provided and O rings 25 serving as a sealing member are also provided. The piston 40 is made of metal, has a shaft member 41, is provided with a first flange 42 on the base end side thereof, and is further provided with a second flange 43 in the vicinity of the first flange 42, as illustrated in FIG. 7. The first flange 42 and the second flange 43 have a disc shape, and have the same diameter. The O rings 25 include one disposed between the first flange 42 and the second flange 43 and one disposed on another side of the second flange 43. A recess portion 44 having a predetermined size is formed in a distal end surface of the shaft member 41. In a state where the piston 40 is disposed in the internal space of the distal end portion 21b before the actuation of the actuator 20, the first flange 42, which serves as a surface receiving pressure of the combustion product from the igniter 22, is exposed on side of the combustion chamber 20a, and the distal end of the shaft member 41 of the piston 40 is inserted into the opening 27.

Then, when the igniter 22 is activated and the combustion product is discharged into the combustion chamber 20a and thus the pressure therein rises, the first flange 42 receives the pressure, resulting in the piston 40 sliding toward the distal end side. Thus, the actuator 20 has a mechanism with the igniter 22 serving as an actuation source and the piston 40 serving as an output unit. Since the second flange 43 has a larger diameter than the opening 27, the distance by which the piston 40 can slide is limited. Thus, the distance by which the shaft member 41 of the piston 40 can protrude from the distal end surface of the distal end portion 21b of the body 21 is limited. Further, the piston 40 may be formed of a resin, and in such case, metal may be used together for a part to which heat resistance and pressure resistance are required.

Additionally, as an alternative mechanism to adjust the pressure applied to the piston 40, the combustion chamber 20a of the actuator 20 may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01/031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

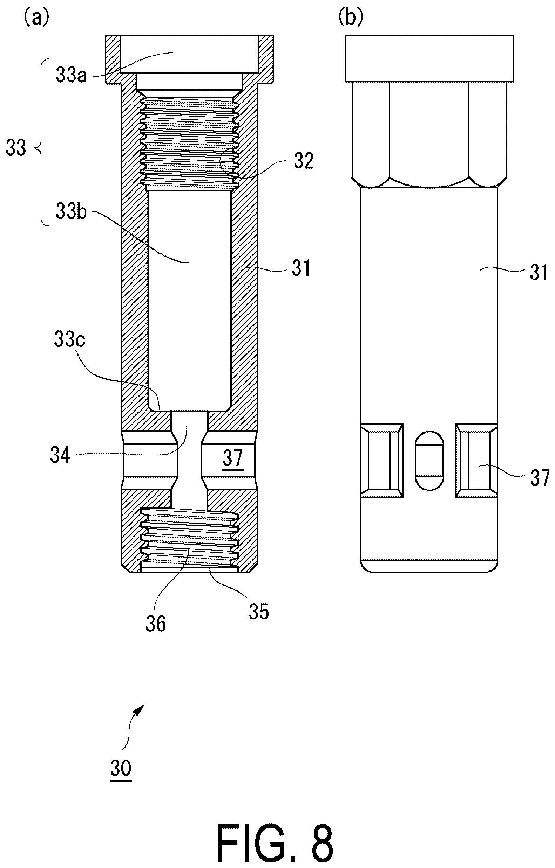

Next, the attachment 30 will be described based on FIG. 8. Note that FIG. 8 includes the diagram (a) on the left side that is a cross-sectional view of the attachment 30, and the diagram (b) on the right side that is an external view of the attachment 30. The attachment 30 is a member for attaching the actuator 20, the plunger 80, and the container 70 as illustrated in FIG. 5. For a body 31 of the attachment 30, nylon 6-12, polyarylate, polybutylene terephthalate, polyphenylene sulphide, a liquid crystal polymer, or the like, which are publicly known, may be used for example. Further, a filler such as glass fibers and glass filler may be contained in those resins. 20 to 80 mass% of glass fibers may be contained in polybutylene terephthalate, 20 to 80 mass% of glass fibers may be contained in polyphenylene sulphide, or 20 to 80 mass% of minerals may be contained in a liquid crystal polymer.

The internal space of the body 31 includes a first region 33, extending from the base end side to the center, where the actuator 20 is disposed as illustrated in FIG. 5. The first region 33 includes a region 33a on the base end side where the base end portion 21c of the actuator 20 is generally positioned, and a region 33b on the distal end side of the first region 33 where the center portion 21a and the distal end portion 21b of the actuator 20 are generally positioned. The region 33b has a smaller diameter than the region 33a. The female thread portion 32 is disposed on the inner wall surface at a portion of the region 33b close to the region 33a. The female thread portion 32 is formed to engage with the male thread portion 26 provided on the center portion 21a of the actuator 20.

The internal space of the body 31 further includes a second region 34 in communication with the first region 33. The second region 34 is a region in which the plunger 80 is generally disposed as illustrated in FIG. 5, and is a hollow region formed in a cylindrical shape extending along the axial direction of the body 31. The second region 34 has one end in communication with the region 33b of the first region 33. The second region 34 has a diameter smaller that is smaller than the diameter of the region 33b, and enables a sliding movement of the plunger 80. A through hole 37 extends from a side outer surface of the attachment 30 to the second region 34, to be formed through the body 31. Through the through hole 37, the user can check the status (such as whether the injector assembly 10 is before or after being actuated, for example) of the plunger 80 in the injector assembly 10 from the outside (see FIG. 1).

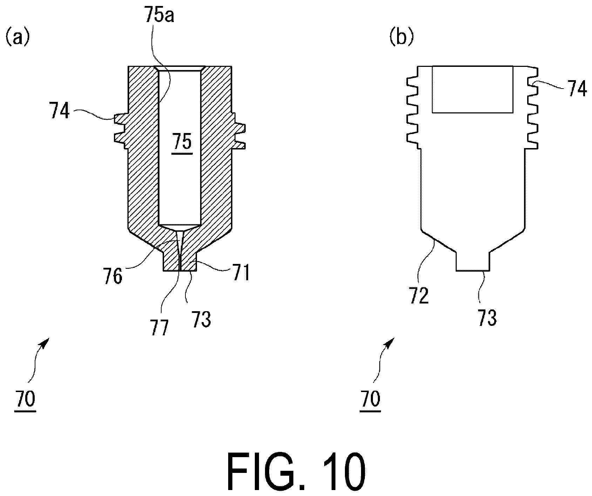

The internal space of the body 31 further includes a third region 35 in communication with the second region 34. The third region 35 is a region in which a part of the container 70 is generally disposed as illustrated in FIG. 5, and has one end in communication with the second region 34, and has the other end open to the distal end surface of the attachment 30. A female thread portion 36 for attachment to the container 70 is formed in the third region 35. The female thread portion 36 is screwed with a male thread portion 74 of the container 70 illustrated in FIG. 10 described below, whereby the attachment 30 and the container 70 are coupled to each other.

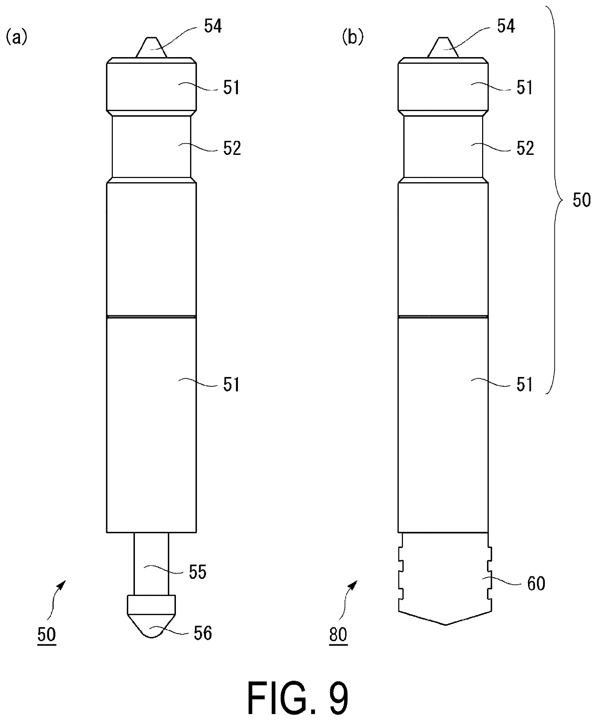

Next, the plunger 80 will be described based on FIG. 9. FIG. 9 includes the diagram (a) on the left side that is an external view of a plunger rod 50, which is one of the components of the plunger 80, and a diagram (b) on the right side that is an external view of the plunger 80. The plunger 80 is a member that pressurizes the ejection solution by energy received from the piston 40, and a resin material suitable for the pressurization (for example, a resin material similar to that used for the attachment 30) can be used for the plunger rod 50. The plunger rod 50 includes a shaft member 51, and has a base end side end surface provided with a protrusion 54. The protrusion 54 is shaped and sized to be capable of fitting in the recess portion 44 of the shaft member of the piston 40 of the actuator 20, when the plunger 80 is incorporated in the injector assembly 10. A reduced diameter portion 52 that has a diameter smaller than other portions of the shaft member 51 is provided in an intermediate portion of the shaft member 51 close to the base end.

Further, in the plunger rod 50, a protrusion 56 is provided to a distal end side of the shaft member 51 with a neck portion 55 with a smaller diameter than the shaft member 51 provided in between. The protrusion 56 is shaped like a weight to have a diameter being greater than the diameter of the neck portion 55 near a portion to be connected with the neck portion 55 and reducing toward the distal end side. The maximum diameter of the protrusion 56 is smaller than the diameter of the shaft member 51. A stopper portion 60 formed of an elastic member such as rubber is attached to the neck portion 55 and the protrusion 56, whereby the plunger 80 is formed (see FIG. 9(b)). An attachment hole (not illustrated) is formed in the stopper portion 60, and engages with the neck portion 55 and the protrusion 56, and the stopper portion 60 is less likely to be detached from the plunger rod 50.

Specific examples of materials of the stopper portion 60 include butyl rubber and silicon rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the stopper portion 60, a polyvinyl chloride-based elastomer, an olefin-based elastomer, a polyester-based elastomer, a polyamide-based elastomer, a polyurethane-based elastomer, various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 60 pressurizes the ejection solution by sliding within the container 70 described below. Thus, a surface of the stopper portion 60 and an inner wall surface 75a of the accommodating space 75 of the container 70 may be coated or processed using various matters, to guarantee/adjust slidability between the stopper portion 60 and the inner wall surface 75a of the accommodating space 75 of the container 70. Examples of the coating agent may include polytetrafluoroethylene (PTFE), silicon oil, diamond-like carbon, nano diamond, and the like.

Next, the container 70 will be described based on FIG. 10. Note that FIG. 10 includes the diagram (a) on the left side that is a cross-sectional view of the container 70, and the diagram (b) on the right side that is an external view of the container 70. The container 70 is a member containing an ejection solution to be pressurized by the plunger 80, and is a member that defines a flow path for injecting the pressurized ejection solution to the target region. In view of this, a resin material (a resin material of the same type as the attachment 30 for example) may be used for forming the container 70.

The container 70 includes an accommodating space 75, in which the stopper portion 60 of the plunger 80 are movable, accommodating the ejection solution, and a nozzle portion 71 including a flow path 76 connecting the accommodating space 75 to the outside of the container 70. The nozzle portion 71 has a columnar outer circumference on the distal end side. Note that in the injector assembly 10, as illustrated in FIG. 5, a positional relationship between the plunger 80 and the container 70 is determined such that the stopper portion 60 of the plunger 80 can slide within the accommodating space 75 in a direction toward the nozzle portion 71 (direction toward the distal end side). The ejection solution is sealed in a space defined by stopper portion 60 of the plunger 80 and the container 70. The flow path of the container 70 opens in a distal end surface 73 of the nozzle portion 71, and the ejection port 77 is formed. Thus, when the plunger 80 slides within the accommodating space 75, the ejection solution accommodated in the accommodating space 75 is pressurized to be ejected from the ejection port 77 through the flow path 76.

The flow path 76 provided in the container 70 has a diameter smaller than the inner diameter of the accommodating space 75. With this configuration, the ejection solution that has been applied with a high pressure is ejected to the outside through the ejection port 77. The male thread portion 74 for attaching the container 70 to the attachment 30 is formed on the base end side of the container 70. The male thread portion 74 is screwed with the female thread portion 36 of the attachment 30.

Note that the profile on the distal end side of the stopper portion 60 of the plunger 80 is shaped to substantially match the profile of the inner wall surface 75a near a portion where the accommodating space 75 and the flow path 76 are connected to each other (the deepest part of the accommodating space 75). With this configuration, a smallest possible gap can be formed between the stopper portion 60 and the inner wall surface 75a of the container 70 when the plunger 80 slides for ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

Now, how the injector assembly 10 is assembled will be described. In a state where the stopper portion 60 of the plunger 80 is inserted to the deepest part of the accommodating space 75 of the container 70, the plunger 80 is retracted with the ejection port 77 of the container 70 in communication with the ejection solution. The stopper portion 60 and the inner wall surface 75a of the accommodating space 75 are suitably in close contact with each other, the retraction action will produce negative pressure in the accommodating space. Thus, the accommodating space 75 can be filled with the ejection solution through the ejection port 77. In this process, the plunger 80 is retracted to an extent enough for making the part of the plunger 80 (plunger rod 50) protruding from the container 70 pass through the second region 34 to reach the first region 33 (the region 33b illustrated in FIG. 8), when the container 70 is attached to the attachment 30 in this state.

After the container 70 filled with ejection solution in the accommodating space 75 is attached to the attachment 30, the actuator 20 is inserted to the attachment 30 from the side of the first region 33. The actuator 20 is inserted until the distal end surface of its distal end portion 21b comes into contact with a distal end surface 33c of the region 33b of the attachment 30 (see FIG. 8). Then, in this process, the male thread portion 26 provided to the center portion 21a of the actuator 20 is screwed with the female thread portion 32 of the attachment 30, whereby the actuator 20 and the attachment 30 are suitably coupled to each other. Furthermore, in this process, the recess portion 44 of the shaft member 41 of the piston 40, which is incorporated in the actuator 20, engages with the protrusion 54 of the shaft member 51 of the plunger 80, and the plunger 80 is pushed by the piston 40 toward the distal end side. Note that, a fixing force of the piston 40 in the distal end portion 21b of the actuator 20 is set to an extent that the piston 40 can slide in the distal end portion 21b in a sufficiently smooth manner by a pressure received from the combustion product produced by the igniter 22, and to an extent that the piston 40 can suitably resist force received from the plunger 80 such that the position of the piston 40 is not displaced when the injector assembly 10 is assembled. Alternatively, a stopper may be formed at an intended position of the piston 40, and the top surface of the first flange 42 of the piston 40 faces the combustion chamber 20a of the actuator 20 and is not displaced toward the combustion chamber 20a as illustrated in FIG. 6.

Thus, when the actuator 20 is attached to the attachment 30 to which the container 70 and plunger 80 are attached as described above, the plunger 80 is pushed to move from the piston 40 toward the distal end side, whereby the plunger 80 is positioned at a predetermined position within the container 70. Note that, in response to pressing of the plunger 80, a part of the ejection solution is discharged from the ejection port 77.

When the plunger 80 is thus positioned at the final position as described above, formation of the injector assembly 10 is completed. In this injector assembly 10, the position of the stopper portion 60 of the plunger 80 in the accommodating space 75 of the container 70 is mechanically determined. The final position of the stopper portion 60 is a position uniquely determined in the injector assembly 10, and hence an amount of the ejection solution that is finally stored in the accommodating space 75 in the injector assembly 10 can be a predetermined amount determined in advance.

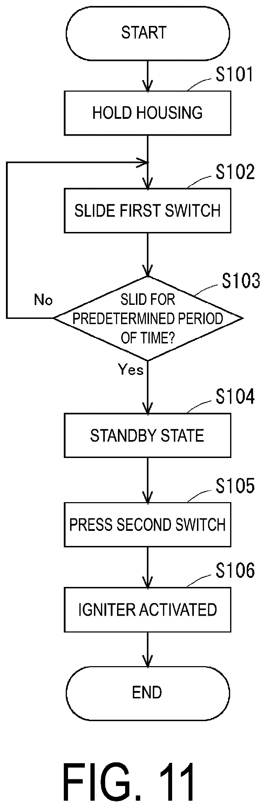

The injector assembly 10 thus configured can be loaded into the housing 2 with the ignition pin 22b of the igniter 22 fitted into the socket 7 on the housing 2, whereby the injector 1 is prepared to be usable (see FIGS. 1 to 3). An operation procedure for the injector 1 in the usable state to eject the ejection solution will be described with reference FIG. 11. First of all, the user holds the housing 2 of the injector 1 with one hand (process in S101). In this process, the fingertip of the user holds the grip portion 2a while resting on the front side of the housing 2. Then, the user moves the portion of the palm covering the first switch 5, that is, a portion of the palm close to the base of the thumb away from the first switch 5, and performs a downward sliding operation on the first switch 5 using the thumb for example (process in S102). In this process, the user maintains the sliding of the first switch 5. Then, in S103, the control unit 90 determines whether the sliding of the first switch has continued for a predetermined period of time (for example, three seconds). The processing proceeds to the processing in S104 when the result of the determination is YES. The processes in S102 and after are performed again without transitioning to the standby state when the result of the determination is NO.

Then, in S104, the control unit 90 places the injector 1 in the standby state. In this process, the injector 1 may issue a predetermined notification with an indicator or the like using sound or an LED, and the user can recognize the standby state of the injector 1. The user then presses the second switch 6 with the ejection port 77 being in contact with the target region (process in S105). As a result, the igniter 22 is activated, and the ejection solution is pressurized via the piston 40 and the plunger 80. Thus, the ejection is implemented, and the ejection solution is injected into the target region (process in S106).

As described above, in the injector 1, the control unit 90 receives operations by the user, different from each other in content, on the first switch 5 and the second switch 6, determines whether the operation is appropriate, and controls the activation of the igniter 22. Furthermore, the switches are configured such that in a series of operations in the injector 1 for ejection of the ejection solution, an erroneous operation on the first switch 5 while the housing 2 is being held can be suppressed, and the second switch 6 is easily recognizable. Thus, the improvement in the safety and the stable operation of the injector 1 can both be favorably achieved.

Each aspect disclosed in the present specification can be combined with any other feature disclosed herein.

### Reference Signs List

1 Injector
2 Housing
2a Grip portion
3 Power cable
4 Connector
5 First switch
6 Second switch
10 Injector assembly
20 Actuator
21 Body
22 Igniter
30 Attachment
31 Body
40 Piston
50 Plunger rod
51 Shaft member
52 Reduced diameter portion
60 Stopper portion
70 Container
71 Nozzle portion
75 Accommodating space
76 Flow path
77 Ejection port
80 Plunger
90 Control unit

## Claims

1. A needleless injector (1) that ejects an intended injection substance to a target region without using an injection needle, the needleless injector (1) comprising:
a housing part that includes an accommodating space (75) in which the intended injection substance is accommodated, and defines a flow path (76) through which the intended injection substance is ejected to the target region;
a driving part that imparts an ejection energy for ejecting the intended injection substance;
a pressurizing unit that, upon being imparted with the ejection energy, pressurizes the intended injection substance accommodated in the accommodating space (75);
an injector enclosure (2) that has a tubular shape, incorporates at least one of the housing part, the driving part, or the pressurizing unit, and is configured to be capable of being held by one hand of a user;
a first switch (5) and a second switch (6) for activation of the driving part, the first switch (5) and the second switch (6) being provided to the injector enclosure (2); and
a control unit (90) that controls the activation of the driving part, via a predetermined operation on the first switch (5) and the second switch (6), wherein
the second switch (6) is arranged on an upper side of the injector enclosure (2) to be able to be pressed, **characterized in that**
the first switch (5) is arranged to be slidable in a predetermined direction different from a contact direction of a palm of the user, in a first region on a side surface that is a part of a surface of the injector enclosure (2) positioned on side of the user, the first region being covered by the palm of the user in a holding state of the user, the contact direction being a direction along which the palm of the user approaches the first switch (5) when attempting to hold the injector enclosure (2) with one hand to cover the first region with the palm, and
the second switch (6) has an upper side surface with at least a part inclined toward the side of the user to be visible from the user in a holding state of the user,.

2. The needleless injector (1) according to claim 1, wherein the second switch (6) is arranged in a second region in the upper side surface of the injector enclosure (2), the second region being configured to be inclined similarly to the at least a part of the upper side surface of the second switch (6), toward the side of the user.

3. The needleless injector (1) according to claim 2, wherein the second switch (6) in the second region is arranged on a virtual straight line (L1) that is an extension, along the surface of the injector enclosure (2), of a slide straight line of the first switch (5) along the predetermined direction, or in vicinity of the virtual straight line.

4. The needleless injector (1) according to any one of claims 1 to 3, wherein the control unit (90) is configured to activate the driving part in response to a predetermined pressing operation on the second switch (6) performed after a predetermined sliding operation on the first switch (5) by the user.

5. The needleless injector (1) according to claim 4, wherein the predetermined sliding operation is an operation of sliding the first switch (5) in the predetermined direction and maintaining the sliding state for a predetermined period of time.

6. The needleless injector (1) according to any one of claims 1 to 5, wherein
the driving part is an ignition device that generates the ejection energy through combustion of an ignition charge upon being supplied with ignition current,
a power cable (3) for supplying the ignition current from a power supply to the ignition device extends from the injector enclosure (2), and
the injector enclosure (2) is configured to have the power cable (3) extending toward a direction away from the user in a holding state of the user with the first switch (5) facing toward the user.

7. The needleless injector (1) according to claim 6, wherein
the injector enclosure (2) has a side surface, on a side opposite to the first switch (5) with the second switch (6) provided in between, provided with a connector (4) to which the power cable (3) is connected, and
the connector (4) is attached to the injector enclosure (2) to make the power cable (3) extend along an inclined surface defined by the at least a part of the upper side surface of the second switch (6).

8. The needleless injector (1) according to claim 7, wherein the connector (4) is configured to enable the power cable (3) to be detachably attached.

## Patentansprüche

1. Ein nadelloser Injektor (1), der eine vorgesehene Injektionssubstanz ohne Verwendung einer Injektionsnadel zu einem Zielbereich ausstößt, wobei der nadellose Injektor (1) umfasst:
einen Gehäuseteil, der einen Aufnahmeraum (75) enthält, in dem die vorgesehene Injektionssubstanz aufgenommen ist, und einen Strömungsweg (76) definiert, durch den die vorgesehene Injektionssubstanz zu dem Zielbereich ausgestoßen wird;
einen Antriebsteil, der eine Ausstoßenergie zum Ausstoßen der vorgesehenen Injektionssubstanz überträgt;
eine Druckbeaufschlagungseinheit, die bei Übertragung der Ausstoßenergie die in dem Aufnahmeraum (75) aufgenommene vorgesehene Injektionssubstanz unter Druck setzt;
eine Injektoreinfassung (2), die eine rohrförmige Gestalt aufweist, mindestens eines von dem Gehäuseteil, dem Antriebsteil oder der Druckbeaufschlagungseinheit enthält und so konfiguriert ist, dass es von einer Hand eines Benutzers gehalten werden kann;
einen ersten Schalter (5) und einen zweiten Schalter (6) zum Aktivieren des Antriebsteils, wobei der erste Schalter (5) und der zweite Schalter (6) an der Injektoreinfassung (2) vorgesehen sind; und
eine Steuereinheit (90), die die Aktivierung des Antriebsteils über eine vorbestimmte Betätigung an dem ersten Schalter (5) und dem zweiten Schalter (6) steuert, wobei
der zweite Schalter (6) an einer Oberseite der Injektoreinfassung (2) angeordnet ist, so dass er gedrückt werden kann, **dadurch gekennzeichnet, dass**
der erste Schalter (5) so angeordnet ist, dass er in einer ersten Region an einer Seitenfläche, die ein Teil einer Oberfläche der Injektoreinfassung (2) ist, die auf der Seite des Benutzers positioniert ist, in einer vorbestimmten Richtung verschiebbar ist, die sich von einer Kontaktrichtung einer Handfläche des Benutzers unterscheidet, wobei der erste Bereich in einem Haltezustand des Benutzers von der Handfläche des Benutzers bedeckt ist, wobei die Kontaktrichtung eine Richtung ist, entlang der sich die Handfläche des Benutzers dem ersten Schalter (5) nähert, wenn er versucht, die Injektoreinfassung (2) mit einer Hand zu halten, um den ersten Bereich mit der Handfläche zu bedecken, und
der zweite Schalter (6) eine Oberseitenfläche mit mindestens einem Teil aufweist, der zur Seite des Benutzers geneigt ist, um für den Benutzer in einem Haltezustand des Benutzers sichtbar zu sein.

2. Der nadellose Injektor (1) nach Anspruch 1, wobei der zweite Schalter (6) in einem zweiten Bereich in der Oberseitenfläche der Injektoreinfassung (2) angeordnet ist, wobei der zweite Bereich so konfiguriert ist, dass er ähnlich wie der mindestens eine Teil der Oberseitenfläche des zweiten Schalters (6) zur Seite des Benutzers hin geneigt ist.

3. Der nadellose Injektor (1) nach Anspruch 2, wobei der zweite Schalter (6) in dem zweiten Bereich auf einer virtuellen Geraden (L1) angeordnet ist, die eine Verlängerung einer Gleitgeraden des ersten Schalters (5) entlang der Oberfläche der Injektoreinfassung (2) entlang der vorbestimmten Richtung, oder in der Nähe der virtuellen Geraden.

4. Der nadellose Injektor (1) nach irgendeinem der Ansprüche 1 bis 3, wobei die Steuereinheit (90) so konfiguriert ist, dass sie den Antriebsteil in Reaktion auf eine vorbestimmte Druckbetätigung auf den zweiten Schalter (6) aktiviert, die nach einer vorbestimmten Gleitbetätigung am ersten Schalter (5) durch den Benutzer ausgeführt wird.

5. Der nadellose Injektor (1) nach Anspruch 4, wobei die vorbestimmte Gleitbetätigung eine Betätigung ist, bei der der erste Schalter (5) in der vorbestimmten Richtung verschoben und der Gleitzustand für eine vorbestimmte Zeitdauer aufrechterhalten wird.

6. Der nadellose Injektor (1) gemäß irgendeinem der Ansprüche 1 bis 5, wobei
der Antriebsteil eine Zündvorrichtung ist, die durch Verbrennung einer Zündladung bei Zufuhr von Zündstrom die Ausstoßenergie erzeugt,
ein Stromkabel (3) zum Zuführen des Zündstroms von einer Energiequelle zu der Zündvorrichtung sich von der Injektoreinfassung (2) erstreckt, und
die Injektoreinfassung (2) so konfiguriert ist, dass sich das Stromkabel (3) in einem Haltezustand des Benutzers, wobei der erste Schalter (5) zum Benutzer weist, in einer Richtung vom Benutzer weg erstreckt.

7. Der nadellose Injektor (1) nach Anspruch 6, wobei
die Injektoreinfassung (2) eine Seitenfläche auf einer dem ersten Schalter (5) gegenüberliegenden Seite mit dem dazwischen vorgesehenen zweiten Schalter (6) aufweist, die mit einem Verbinder (4) versehen ist, mit dem das Stromkabel (3) verbunden ist, und
der Verbinder (4) an der Injektoreinfassung (2) angebracht ist, so dass sich das Stromkabel (3) entlang einer geneigten Fläche erstreckt, die durch mindestens einen Teil der Oberseitenfläche des zweiten Schalters (6) definiert ist.

8. Der nadellose Injektor (1) nach Anspruch 7, wobei der Verbinder (4) so konfiguriert ist, dass er ermöglicht, dass das Stromkabel (3) abnehmbar angebracht ist.

## Revendications

1. Injecteur sans aiguille (1) qui éjecte une substance d'injection prévue au niveau d'une région cible sans utiliser d'aiguille d'injection, l'injecteur sans aiguille (1) comprenant :
une partie logement qui inclut un espace d'accueil (75) dans lequel la substance d'injection prévue est logée, et définit un trajet d'écoulement (76) à travers lequel la substance d'injection prévue est éjectée dans la région cible ;
une partie d'entraînement qui confère une énergie d'éjection pour éjecter la substance d'injection prévue ;
une unité de pressurisation qui, lorsqu'il lui est conféré de l'énergie d'éjection, met sous pression la substance d'injection prévue logée dans l'espace d'accueil (75) ;
une enceinte d'injecteur (2) qui présente une forme tubulaire, qui incorpore au moins l'une de la partie logement, de la partie d'entraînement, ou de l'unité de pressurisation, et qui est configurée pour être capable d'être tenue par une main d'un utilisateur ;
un premier commutateur (5) et un second commutateur (6) pour l'activation de la partie d'entraînement, le premier commutateur (5) et le second commutateur (6) étant fournis au niveau de l'enceinte d'injecteur (2) ; et
une unité de commande (90) qui commande l'activation de la partie d'entraînement, par l'intermédiaire d'une opération prédéterminée sur le premier commutateur (5) et le second commutateur (6), dans lequel
le second commutateur (6) est agencé sur un côté supérieur de l'enceinte d'injecteur (2) pour être capable d'être pressé, **caractérisé en ce que**
le premier commutateur (5) est agencé pour pouvoir coulisser dans une direction prédéterminée différente d'une direction de contact d'une paume de l'utilisateur, dans une première région sur une surface latérale qui est une partie d'une surface de l'enceinte d'injecteur (2) positionnée sur un côté de l'utilisateur, la première région étant recouverte de la paume de l'utilisateur sous un état de retenue de l'utilisateur, la direction de contact étant une direction le long de laquelle la paume de l'utilisateur approche le premier commutateur (5) lors de la tentative de tenir l'enceinte d'injecteur (2) avec une main pour couvrir la première région avec la paume, et
le second commutateur (6) présente une surface latérale supérieure avec au moins une partie inclinée vers le côté de l'utilisateur pour être visible depuis l'utilisateur sous un état de retenue de l'utilisateur.

2. Injecteur sans aiguille (1) selon la revendication 1, dans lequel le second commutateur (6) est agencé dans une seconde région dans la surface latérale supérieure de l'enceinte d'injecteur (2), la seconde région étant configurée pour être inclinée de manière similaire à la au moins une partie de la surface latérale supérieure du second commutateur (6), vers le côté de l'utilisateur.

3. Injecteur sans aiguille (1) selon la revendication 2, dans lequel le second commutateur (6) dans la seconde région est agencé sur une ligne droite virtuelle (L1) qui est une extension, le long de la surface de l'enceinte d'injecteur (2), d'une ligne droite de coulissement du premier commutateur (5) le long de la direction prédéterminée, ou à proximité de la ligne droite virtuelle.

4. Injecteur sans aiguille (1) selon l'une quelconque des revendications 1 à 3, dans lequel l'unité de commande (90) est configurée pour activer la partie d'entraînement en réponse à une opération de pressage prédéterminée sur le second commutateur (6) exécutée après une opération de coulissement prédéterminée sur le premier commutateur (5) par l'utilisateur.

5. Injecteur sans aiguille (1) selon la revendication 4, dans lequel l'opération de coulissement prédéterminée est une opération de coulissement du premier commutateur (5) dans la direction prédéterminée et de maintien de l'état de coulissement sur une période de temps prédéterminée.

6. Injecteur sans aiguille (1) selon l'une quelconque des revendications 1 à 5, dans lequel
la partie d'entraînement est un dispositif d'ignition qui génère de l'énergie d'éjection à travers une combustion d'une charge d'ignition lorsqu'elle est alimentée d'un courant d'ignition,
un câble d'alimentation (3) pour alimenter le courant d'ignition depuis une alimentation d'énergie au dispositif d'ignition s'étend depuis l'enceinte d'injecteur (2), et
l'enceinte d'injecteur (2) est configurée pour avoir le câble d'alimentation (3) s'étendant vers une direction à l'opposé de l'utilisateur sous un état de retenue de l'utilisateur avec le premier commutateur (5) faisant face vers l'utilisateur.

7. Injecteur sans aiguille (1) selon la revendication 6, dans lequel
l'enceinte d'injecteur (2) présente une surface latérale, sur un côté opposé au premier commutateur (5) avec le second commutateur (6) fourni entre eux, pourvue d'un connecteur (4) auquel le câble d'alimentation (3) est connecté, et
le connecteur (4) est fixé à l'enceinte d'injecteur (2) pour permettre au câble d'alimentation (3) de s'étendre le long d'une surface inclinée définie par la au moins une partie de la surface latérale supérieure du second commutateur (6).

8. Injecteur sans aiguille (1) selon la revendication 7, dans lequel le connecteur (4) est configuré pour permettre au câble d'alimentation (3) d'être fixé de manière détachable.
